# EUROPÄISCHE PATENTSCHRIFT

(11) **EP 0 555 514 B1**
(45) Veröffentlichungstag und Bekanntmachung des Hinweises auf die Patenterteilung: **30.09.1998**
(21) Anmeldenummer: 92107579.2
(22) Anmeldetag: 05.05.1992
(51) Int. Cl.: C11D 17/04, B65D 21/02, B65D 25/22

(54) **Wasch-, Reinigungs-, Desinfektions- und/oder Konservierungsmittel enthaltender Mehrwegbehälter**
Re-usable receptacle containing washing, cleaning, desinfecting and/or preserving agents
Récipients réutilisable contenant des produits de nettoyage, de désinfection et/ou de conservation

(30) Priorität: 14.02.1992 DE 4204489
(43) Veröffentlichungstag der Anmeldung: 18.08.1993
(73) Patentinhaber: ECOSAN Hygiene GmbH, 63450 Hanau (DE)
(72) Erfinder: Mandler, Gunter, W-6301 Heuchelheim (DE); Rieber, Wolfram, Dr. rer. nat. Dipl.-Chem., W-6708 Neuhofen (DE); Sander, Richard, Dr.-Ing. Dipl.-Chem., W-7507 Pfinztal-Berghausen (DE); Kompan, Jürgen, W-6725 Römerberg 2 (DE)
(74) Vertreter: Müller-Boré & Partner Patentanwälte

(56) Entgegenhaltungen:
- EP-A- 0 225 859
- EP-A- 0 242 966
- EP-A- 0 375 022
- WO-A-89/11753
- WO-A-92/12062
- DE-A- 2 851 605
- US-A- 4 808 236
- DATABASE WPI Derwent Publications Ltd., London, GB; AN 89-224560 & JP-A-1 161 100 (LION CORP.) 23. Juni 1989

## Beschreibung

Die Erfindung betrifft den in den Patentansprüchen angegebenen Gegenstand. Insbesondere betrifft die Erfindung ein Verfahren zum Herstellen verpreßter, geformter und wenigstens teilweise porös ausgebildeter Wasch-, Reinigungs-, Desinfektions- und/oder Konservierungsmittel ohne Verwendung einer vorerwärmten Schmelze oder Lösung, bei dem eine vorgefertigte Vormischung aus pulver- und/oder granulatförmigen Wirkstoffen mit oder ohne Vermischen dieser vorgefertigten Mischung mit einem Bindemittel hergestellt, in einen geeignet geformten Mehrweg-Behälter einfüllt und unter Druck verdichtet wird, so daß ein in dem Behälter verfestigter Kompaktblock mit Pulverstruktur gebildet wird.

Die Erfindung betrifft ebenfalls wiederverwendbare bzw. recyclebare, insbesondere zum Einsatz in Waschmaschinen geeignete Wasch-, Reinigungs-, Desinfektions- und/oder Konservierungsmittel enthaltende Mehrweg-Behälter, die derart gestaltet sind, daß Wasch-, Reinigungs-, Desinfektions- und/oder Konservierungsmittel aus dem Behälter abgegeben wird, wenn ein Strom wäßriger Flüssigkeit auf eine diesem ausgesetzte Oberfläche des Wasch-, Reinigungs-, Desinfektions- und/oder Konservierungsmittels gegeben wird. Die Erfindung betrifft schließlich die Verwendung dieser Mehrweg-Behälter.

In den Unteransprüchen sind vorteilhafte Ausführungsformen der Erfindung enthalten.

Neben flüssigen, pastösen oder pulverförmigen Detergentien sind auch geformte, stückförmige, feste Detergentienprodukte seit langem bekannt und in Gebrauch, wie z. B. Seifenstücke, Syndetseifenstücke, Toilettenkegel oder -tabletten, geformte Detergentien für Spül- und Waschmaschinen usw..

Eine allgemeine Ubersicht ist z. B. in der Publikation von H. E. Tschakert, Seifen, Öle, Fette, Wachse, 98, (1972), 793 - 801, 845 - 849 und ibid. 99 (1973), 3 - 7 enthalten.

Neben der Herstellung derartiger geformter stückförmiger Detergentienprodukte durch Preß- und Extrudiervorgänge (zu Tabletten, Briketts und dergleichen) ist seit langem die Herstellung durch Eingießen einer zur Verfestigung befähigten Lösung oder Schmelze in Formen gebräuchlich, was gegenüber dem Pressen den Vorteil bringt, daß man auch komplizierte und z.B. unregelmäßig aufgebaute Formstücke auf einfache Weise herstellen kann.

Häufig wird in diesem Falle die Lösung oder Schmelze in erwärmtem Zustand in die Formen gegossen und verfestigt sich beim Erkalten.

Die Formstücke können nach der Verfestigung entweder aus den Gießformen herausgenommen werden und in gesonderter Verpackung zum Verwender gelangen, oder aber das als Gießform verwendete Behältnis dient gleichzeitig als Verpackung für das geformte Detergentienstück und gelangt mit diesem verbunden zum Verwender, wobei in der Regel bei jeder Anwendung die für den jeweiligen Verwendungszweck erforderliche Detergentienmenge aus dem geformten stückförmigen Detergentienprodukt in seiner Umhüllung durch Einwirken von entsprechenden Lösungsmitteln, meist Wasser, herausgelöst wird.

Beispiele für derartige Produktformulierungen und Herstellungsverfahren sind z. B. in Tenside 8 (1991), 275, in Tenside 11 (1974), 330, in Seife, Öle, Fette, Wachse 96, Nr. 23 (1970), 823 sowie insbesondere in der bereits oben erwähnten Publikation von H. E. Tschakert aufgeführt.

Desgleichen bringt das dem einschlägigen Fachmann bestens vertraute "Jahrbuch für den Praktiker", Verlag für Chem. Ind. Ziolkowski, Augsburg, 1972, S. 194, 1973, S. 229, 1974, S. 110 und S. 132, 134, 135, 1975, S. 116, 117, 118, 1976, S. 116 - 120 verschiedene Formulierungsbeispiele für geformte Detergentienstücke, die durch Gießen einer erwärmten Schmelze bzw. Lösung in Formen und Erhärten beim Erkalten hergestellt werden.

Auch in jüngerer Zeit hat sich die Patentliteratur mit dem Verfahren der Herstellung von geformten festen Reinigungsmitteln durch Einfüllen einer erwärmten Schmelze bzw. Lösung in Formen und Verfestigen beim Erkalten befaßt, so z. B. die Eur. Pat. Anm. 0 003 769, die Eur. Pat. Anm. 0 307 587, Aufgießen von alkalischer wässeriger Lösung, sowie die deutschen Patentanmeldungen DE 35 19 353, DE 35 19 354, DE 35 19 355, DE 36 34 812.

Alle diese publizierten Herstellungsverfahren für feste, geformte Detergentienkörper sind mit produktionstechnischen oder qualitativen Nachteilen behaftet, da
a) im Falle der Herstellung durch Tablettieren, Brikettieren etc. nur bestimmte einfache Formen zu erzeugen sind und außerdem sehr aufwendige Anlagen zur Verarbeitung erforderlich sind;
b) im Falle der Herstellung durch Gießen einer Schmelze oder erwärmten Lösung in eine Form beträchtliche Energie zum Schmelzen und Warmhalten der Ausgangsmischungen erforderlich ist und außerdem thermisch empfindliche Rezepturbestandteile infolge des zwangsweise längere Zeit andauernden Verweilens bei höherer Temperatur eine höhere Schädigung bzw. einen höheren Abbau- oder Zersetzungsgrad erleiden, als wenn gar keine oder nur eine sehr kurzfristige Erwärmung stattfinden würde.

Die EP-A-0242966 zeigt das Verfestigen einer körnigen Detergensmischung in einem Behälter durch das Aufgießen einer erhitzten wäßrigen Lösung, welche dann von oben nach unten die Zwischenräume füllt, ohne daß gerührt wird.

Diese Arbeitsweise hat den Nachteil, daß leicht beim ersten Kontakt der Tränkflüssigkeit mit dem Pulvergemisch ein zu schnelles Verfestigen des oberen Teils in der Mischung im Behälter erfolgt, so daß die Flüssigkeit die unteren Teile der Mischung, insbesondere wenn durch Abrieb Feinteile vorliegen, nicht oder nicht genügend durchtränkt und auf diese Weise eine unvollständige Blockbildung entsteht.

EP-A-0 375 022 beschreibt die Herstellung eines festen Reinigungsmittelblocks, der ausgehend von einem geeigneten granulatförmigen Produkt in einer geeigneten Form mit einem Druck von 3 bis 30 kg/cm², d.h. von 3 × 10⁷ bis 3 × 10⁸Pa (300 at) verpreßt wird. Dabei wird gemäß Beispiel 1 in einem ersten Schritt mit 530 at verpreßt und dann mit 640 at endverpreßt. Die hergestellten Reinigungsmitteltabletten werden aus der Form herausgenommen, verpackt und können dann beim Verbraucher eingesetzt werden.

Die vorliegende Erfindung hat sich daher die Aufgabe gestellt, ein Verfahren zur Herstellung verpreßter, geformter und wenigstens teilweise porös ausgebildeter Wasch-, Reinigungs-, Desinfektions- und/oder Konservierungsmittel- Blöcke zu entwickeln, ohne einen starken Preßvorgang einbeziehen zu müssen und ohne ein Rohstoffgemisch oder Teile davon vor dem Einfüllen in eine Form durch thermische Energie schmelzen oder lösen zu müssen, wobei dennoch ein kompakter Block entsteht, bei welchem die Pulver struktur noch erkennbar ist.

Weiterhin soll ein recyclebarer, insbesondere zum Einsatz in Spül- und Waschmaschinen geeigneter Wasch-, Reinigungs-, Desinfektions- und/oder Konservierungsmittel enthaltender Mehrweg-Behälter zur Verfügung gestellt werden, der derart ausgestaltet ist, daß Wasch-, Reinigungs-, Desinfektions- und/oder Konservierungsmittel aus diesem Behälter abgegeben wird, wenn ein Strom wäßriger Flüssigkeit auf eine diesem ausgesetzte Oberfläche des Wasch-, Reinigungs-, Desinfektions- und/oder Konservierungsmittels abgegeben wird. Dieser Waschmittel-enthaltende Behälter soll weiterhin in ein an diesem Behälter angepaßtes Dosiergerät zum Auflösen dieser Mittel eingesetzt werden können.

Diese Aufgaben werden durch die kennzeichnenden Teile der Ansprüche 1, 2, bzw. 18, bzw. 23 gelöst.

In den Unteransprüchen sind vorteilhafte Ausgestaltungsformen der Erfindung enthalten.

Es wurde überraschenderweise festgestellt, daß man einen verfestigten, kompakten, jedoch nichtschmelzgegossenen Wasch-, Reinigungs-, Desinfektions- und/oder Konservierungsmittel- Block herstellen kann, bei welchem die Pulverstruktur noch erkennbar ist, wenn man eine vorgefertigte Vormischung aus pulver- und/oder granulatförmigen Wirk- und Hilfssubstanzen mit einem geeigneten wasserlöslichen, bei der Herstelltemperatur flüssigen Bindemittel in einer geeigneten Mischeinrichtung, vorzugsweise einem Durchlaufmischer vermischt, so daß eine befeuchtete, aber noch schüttfähige Pulvermasse entsteht, diese Pulvermasse, ggf. auch ohne vorherigen Zusatz von Bindemittel, unter Anwendung von Druck - ohne jedoch stark zu pressen - verdichtet, so daß durch die nachfolgende Verfestigung ein kompakter Block entsteht, bei welchem die Pulverstruktur noch erkennbar ist. Der vorerwähnte Zusatz von Bindemittel ist nicht in jedem Fall obligat, da die eingesetzten Wirksubstanzen Kristallwasser enthalten, das ausreichen kann, um eine befeuchtete aber noch schüttfähige Pulvermasse entstehen zu lassen.

Eine besonders bevorzugte Ausführungsform des erfindungsgemäßen Verfahrens besteht darin, daß eine vorgefertigte Vormischung von pulver- und/oder granulatförmigen Substanzen (Wirkstoffen) mit einem geeigneten wasserlöslichen, bei der Herstelltemperatur flüssigen Bindemittel in einer Menge zwischen 1 und 29 Masse-%, bezogen auf die Gesamtmischung in einer geeigneten Mischeinrichtung vorzugsweise einem Durchlaufmischer vermischt wird, so daß eine "befeuchtete", aber noch schüttfähige Pulvermasse entsteht, diese in das Behältnis eingefüllt und unter Anwendung von Druck d.h. von 1x10⁴ bis 1x10⁶ Pa (0,1 bis 10 at) mittels eines Druckstempels mit gewünschter Oberflächenausgestaltung (eben oder kalottenförmig) verdichtet (jedoch nicht stark gepreßt) wird, wobei durch nachfolgende Verfestigung ein kompakter (jedoch nicht schmelzgegossener) Block entsteht, bei welchem die Pulverstruktur noch erkennbar ist. Dieser Block kann zusätzlich mit einer aufgegossenen Lösung oder Schmelze einer wasserlöslichen Substanz "versiegelt" werden, so daß eine geschlossene, glatte Oberfläche entsteht. Diese Versiegelungsschicht muß im Gegensatz zu der weiter unten beschriebenen Masse keine eigene mechanische Festigkeit aufweisen, sondern dient mehr der optischen Gestaltung und ggf. dem Schutz vor schädlichen Hauteinflüssen bei Berührung der Oberfläche.

Das beschriebene Bindemittel wird aus der Gruppe bestehend aus einer wäßrigen Lösung oder Dispersion eines oder mehrerer wasserlöslicher Salze oder einer organischen Substanz oder auch einer fließfähigen Schmelze oder Gels ausgewählt. Das Bindemittel selbst kann, aber muß nicht obligatorisch bei Temperaturen zwischen 0 und 40°C selbst kristallisationsfähig sein. Das Bindemittel wird in der Regel in Anteilen von 1 bis 29 Masse-%, vorzugsweise 2 bis 10 Masse-% und besonders bevorzugt 3 bis 8 Masse-%, bezogen auf die fertige Gesamtmischung, eingesetzt.

Das Bindemittel kann, aber muß nicht, selbst reinigungsaktiv wirkende Bestandteile enthalten. Besonders geeignet ist eine wäßrige 30 bis 60 gew.-%ige Lösung von Alkali-(ortho-, pyro- oder poly)phosphaten.

Die beschriebene Versiegelungsschicht kann mit einer Gußmasse, die eine gleiche oder eine zu dem Bindemittel verschiedene Zusammensetzung aufweist, erzielt werden. Im Gegensatz zum Bindemittel muß diese Gußmasse jedoch bei Temperaturen zwischen 0 und 40°C fest oder zumindest gelförmig sein. Der Anteil der Gußmasse, bezogen auf den gesamten Füllinhalt, liegt zwischen 0 (bei Verzicht auf Versiegelung) und maximal 20 %.

Als Gußmasse zur Herstellung einer Halteschicht kann vorzugsweise ein bei 0 bis 40°C festes Polyethylenglykol oder ein endgruppenverschlossenes Derivat desselben oder eine kristallisationsfähige, sich bei 0 bis 40°C verfestigende Lösung oder Schmelze von einem oder mehreren Salz(en) wie Soda, Natriumsulfat, Alkalipolyphosphat oder Säuren wie Zitronensäure oder Mischungen dieser Komponenten verwendet werden.

Insbesondere kann man als Versiegelungsschicht eine Mischung aus 30 bis 50 Masse-% Wasser sowie 20 bis 50 Masse-% Natriumpolyphosphat, und/oder 20 bis 50 Masse-% Soda und/oder 20 bis 50 Masse-% Natriumsulfat einsetzen, wobei entweder nur ein oder eine Kombination von zwei oder drei geeigneten Salz(en) enthalten ist (sind) und der Feststoffgehalt zwischen 50 und 70 Masse-% liegt. In einer besonders bevorzugten Ausführungsform kann die Versiegelungsschicht aus einer Mischung aus 50 Masse-% Wasser, 25 Masse-% Natriumpolyphosphat und 25 Masse-% Soda bestehen.

Die üblicherweise angebotenen Grundstoffe für die Wasch-, Reinigungs-, Desinfektions- und Konservierungsmittel liegen in Pulver- oder Granulatform vor. Die Einzelpartikel der Detergentienwirk- und -hilfsstoffe haben einen bevorzugten Durchmesser zwischen 0,01 bis 3 mm. Die Teilchengröße der für die Pulvermischung verwendeten Komponenten ist nicht besonders kritisch.

Insbesondere bei Komponenten, die in technischer Qualität in brauchbaren Korngrößen, z.B. ca. 0,05 bis ca. 1,0 mm lieferbar sind, wird man solche Korngrößen unmittelbar verwenden. Die Verwendung von Pulvern mit viel Feinanteilen, also unterhalb von einem oder einigen Zehntel Millimetern, ist beim vorliegenden Verfahren nicht nachteilig.

Die durch das erfindungsgemäße Verfahren hergestellten, in dem Behälter verfestigten Blöcke können zum Waschen, Reinigen, Spülen sowie zum Desinfizieren bzw. zur antimikrobiellen Behandlung, bzw. zur desodorierenden Behandlung von Wasserkreisläufen und wasserführenden Systemen verwendet werden.

Das erfindungsgemäße Verfahren wird nun durch die folgenden Beispiele näher erläutert.

### Beispiel 1:

a) Es wurden in einem Chargenmischer Pulvervormischungen folgender Zusammensetzung hergestellt (jeweils in Gew.-%):

| | A | B | C | D |
|---|---|---|---|---|
| Natriumtripolyphosphat | 44 | 29 | 55 | 10 |
| Ätznatron-Perlen | 40 | -- | 18 | -- |
| Soda (Pulver) | 12 | 15 | -- | 85 |
| Natriumdichloroisocyanurat . 2 H₂O | 3 | 5 | 2 | -- |
| Natriummetasilikat (wasserfrei) | -- | 50 | 25 | -- |
| nichtionisches Tensid (Lutensol ® LF 131) | 1 | 1 | -- | 5. |

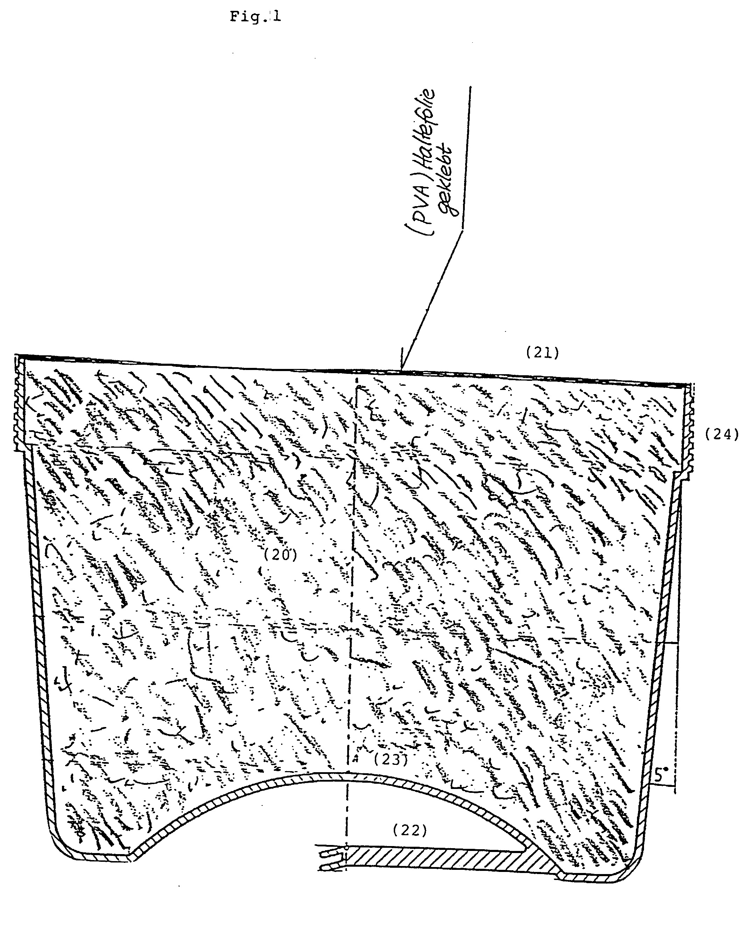

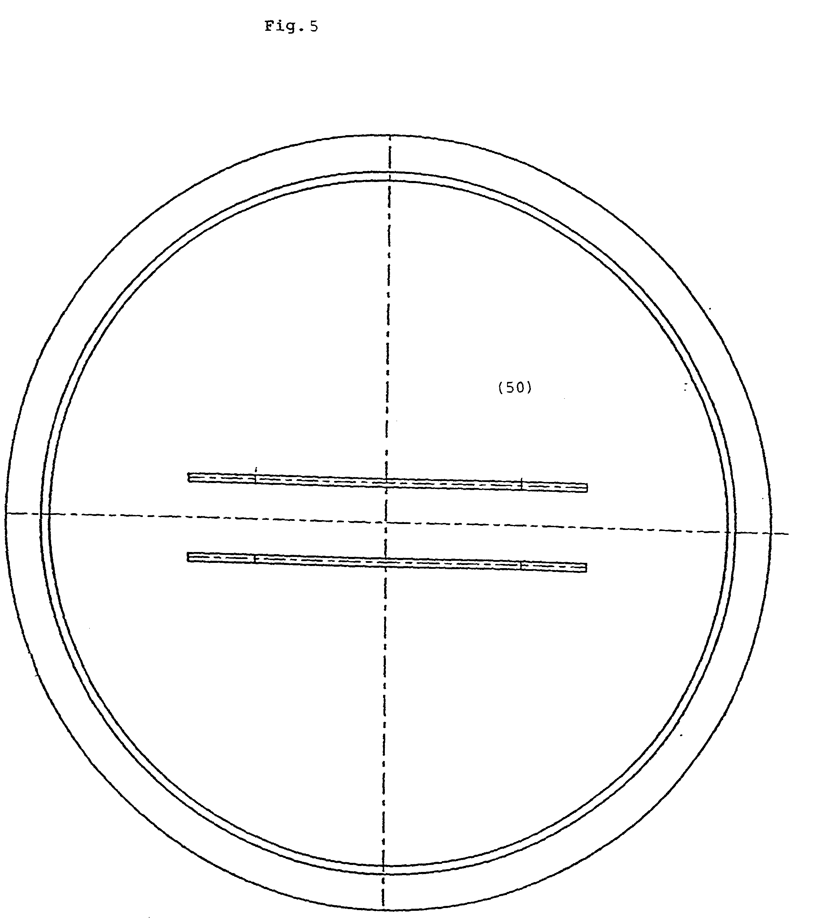

b) Es wurde eine Bindermischung bestehend aus 50 % Wasser und 50 % Natriumpolyphosphat hergestellt und auf eine Temperatur von 20°C gebracht.
c) Jeweils 92 Masse-% der jeweiligen Pulvermischung A bis D wurden mit je 8 Masse-% intensiv vermischt, wobei schon während des Mischens eine Erhöhung der Temperatur beobachtet wurde, die dann nachfolgend auf 65 bis 70°C anstieg. Die resultierenden feuchten, aber schüttfähigen Pulvermassen wurden randvoll in die Behälter 20; 30; 40; 50 (vgl. Fig. 1 bis 5) eingebracht (2,5 Liter) und unter Anwendung eines geringfügigen Drucks mit einem glatten Stempel mit ebener Oberfläche zusammengedrückt. Sodann wurden die Behälter mit einem Schraubdeckel verschlossen und bei Raumtemperatur (20 bis 25°C) gelagert. Die Kontrolle nach 6 Stunden zeigte, daß in den Behältern eine kompakte Masse entstanden war.

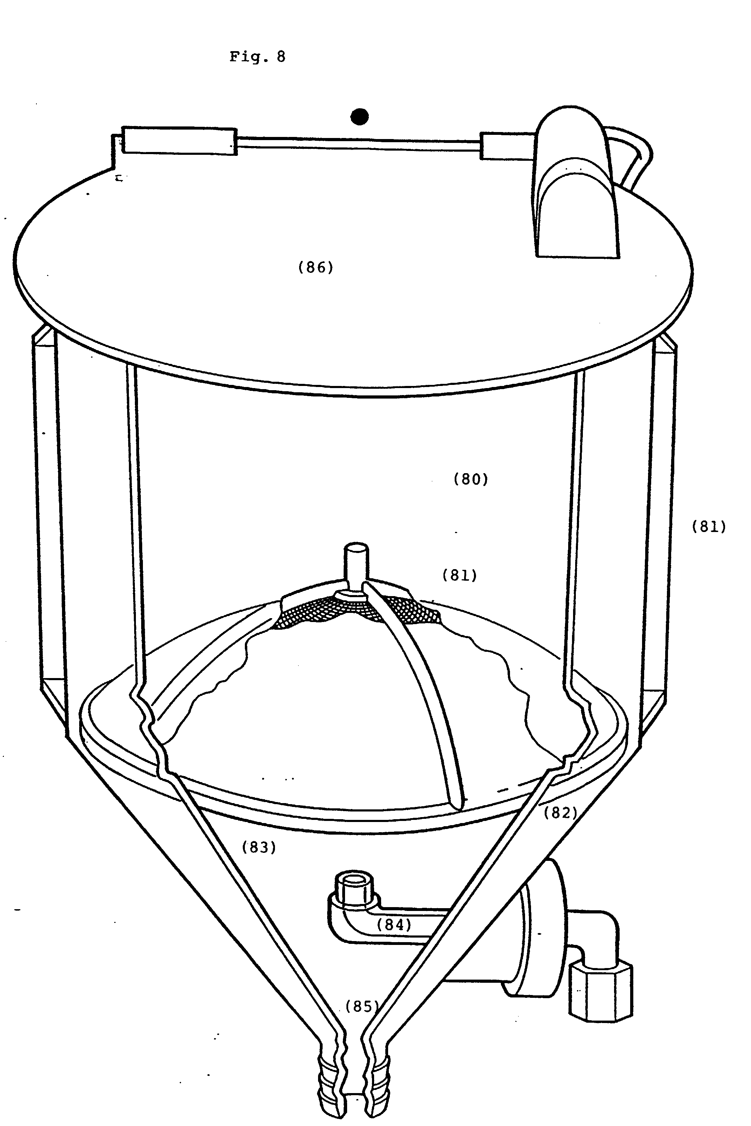

Die Behälter ließen sich mit der Öffnung nach unten in die mit einer Siebeinlage 81 versehenen Einspülvorrichtung 80 (vgl. Fig.8) einsetzen, ohne daß pulver- oder granulatförmige Bestandteile abbröckelten und herausfielen. Durch Besprühen mit Wasser aus einer Düse 84 unterhalb des Siebes 81 ließ sich der gesamte Behälterinhalt problemlos herauslösen. Die entleerten Gebinde enthielten nach Beendigung der Ausspülphase keinerlei sichtbare Rückstände.

### Beispiel 2

In einer zweiten Versuchserie wurden die gleichen Mischungen, wie unter la) bis c) beschrieben, hergestellt und in die Behältnisse gefüllt. Nach dem Zusammendrücken mit dem Stempel wurden jeweils 100 ml einer auf 60°C erwärmten dünnflüssigen Mischung aus 40 Masse-% Wasser , 40 Masse-% Natriumpolyphosphat und 20 Masse-% Soda aufgegossen und diese Mischung verteilte sich dann selbständig gleichmäßig.

Die Behälter wurden verschraubt und bei Raumtemperatur stehengelassen. Nach 6 Stunden Standzeit wurden die Deckel entfernt und die Füllmassen kontrolliert. Wie unter 1 beschrieben, zeigte sich auch hier eine druckfeste Masse, welche jedoch eine gleichmäßig glatte, geschlossene feste Oberfläche aufwies. Auch diese Behältnisse ließen sich problemlos in die Einspülvorrichtung 80 mit der Öffnung nach unten einsetzen und rückstandsfrei durch Besprühen mit Wasser ausspülen.

### Beispiel 3

In einer dritten Versuchsserie wurde die folgende Pulververmischung hergestellt:
- Natriumhydrogensulfat-Pulver 60 Gew.-%
- zitronensäure-monohydrat-Pulver 10 Gew.-%
- Natriumsulfat (wasserfrei)-Pulver 29,8 Gew.-%
- nichtionisches Tensid (Pulver) 0,1 Gew.-% (Talgfettsäurealkohol mit 25 Mol Ethylenoxid)
- Duftstoff Zitrone 0,1 Gew.-%.

Diese Mischung wurde dann wie in Beispiel 1b und 1c näher erläutert ist behandelt,in die Behältnisse gefüllt und nach dem Zusammendrücken mit dem Stempel wurde der Pulverblock mit einer Masse, wie sie in Beispiel 2 beschrieben ist, versiegelt.

Die Behälter wurden verschraubt und bei Raumtemperatur stehengelassen. Nach 6 Stunden Standzeit wurden die Deckelentfernt und die Füllmassen kontrolliert. Wie unter 1. beschrieben, zeigte sich auch hier eine druckfeste Masse, welche jedoch eine gleichmäßig glatte, geschlossene feste Oberfläche aufwies. Auch diese Behältnisse ließen sich problemlos in die Einspülvorrichtung 80 mit der Öffnung nach unten einsetzen und rückstandsfrei durch Besprühen mit Wasser ausspülen.

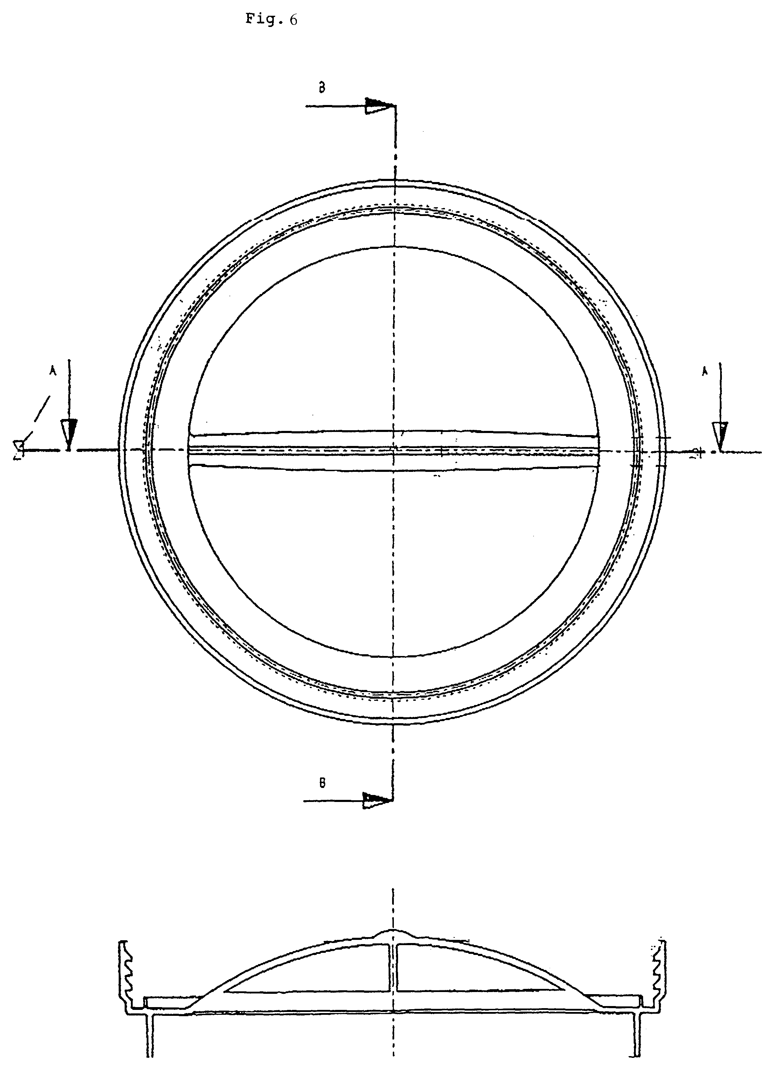

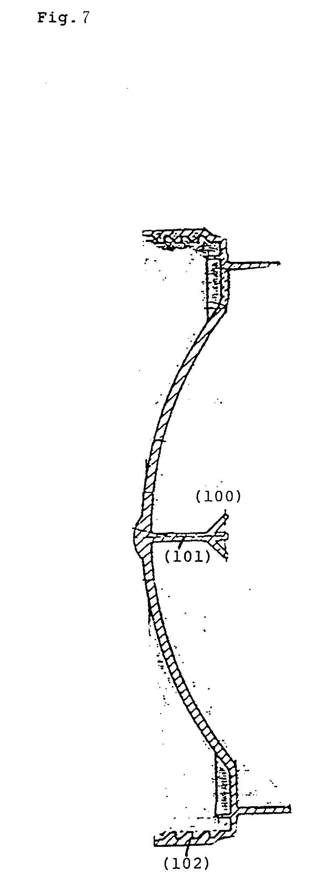

Die erfindungsgemäßen recyclebaren Mehrweg-Behälter sowie deren Abdeckungen sind in den Fig. 1 bis 7 dargestellt.

Es zeigen:
- -Fig. 1: zeigt einen Querschnitt eines randvoll mit Wasch-, Reinigungs-, Desinfektions- und/oder Konservierungsmittel gefüllten erfindungsgemäßen Behälters, der mit einer Folie (beispielsweise PVA- oder PE-Haltefolie) verschlossen ist;
- -Fig. 2: zeigt einen Querschnitt eines erfindungsgemäßen Behälters, der randvoll mit Wasch-, Reinigungs-, Desinfektions- und/oder Konservierungsmittel (mit oder ohne Zusatz von Bindemittel) gefüllt ist und auf den eine Halteschicht aufgeschmolzen worden ist;
- -Fig. 3: zeigt einen Querschnitt eines erfindungsgemäßen Mehrwegbehälter, in den das Wasch-, Reinigungs-, Desinfektions- und/oder Konservierungs- mittelprodukt (ohne Zusatz von Bindemittel) abgefüllt ist und auf den ein wasserlösliches PVA-Formteil aufgebracht ist und der anschließend mittels einer kalottenförmigen Presse verdichtet worden ist;
- -Fig. 4: zeigt einen Querschnitt durch eine erfindungsgemäße Abdeckung aus Polyethylen für einen erfindungsgemäßen Mehrweg-Behälter;
- -Fig. 5: zeigt eine Draufsicht auf einen Deckel gemäß Fig. 4;
- -Fig. 6: zeigt eine besondere Ausführungsform eines vertieften Deckels, der gegebenenfalls kalottenförmig ausgestaltet sein kann, und
- -Fig. 7: zeigt ebenfalls eine andere Ausführungsform eines vertieften, insbesondere kalottenförmigen Deckels mit Haltegriff.

Außerdem, zeigt Fig. 8 die in den Beispielen verwendete Einspülvorrichtung.

Die in Fig. 1 bis 3 dargestellten erfindungsgemäßen, recyclebaren Behälter 20; 30; 40; sind nach oben offen und erweitern sich konisch nach oben. Sie sind für flüssige, feste bzw. pulverförmige Wasch-, Reinigungs-, Desinfektions- und/oder Konservierungsmittel bestimmt. Die Behälter 20; 30; 40; sind aus 2 mm starkem Polyethylen (HD) als Spritzgußteil hergestellt und mit einer Stapelschräge von 5° versehen worden. Sie sind völlig zur Einfüll- und Dosieröffnung ohne Hinterschneidungen geöffnet.

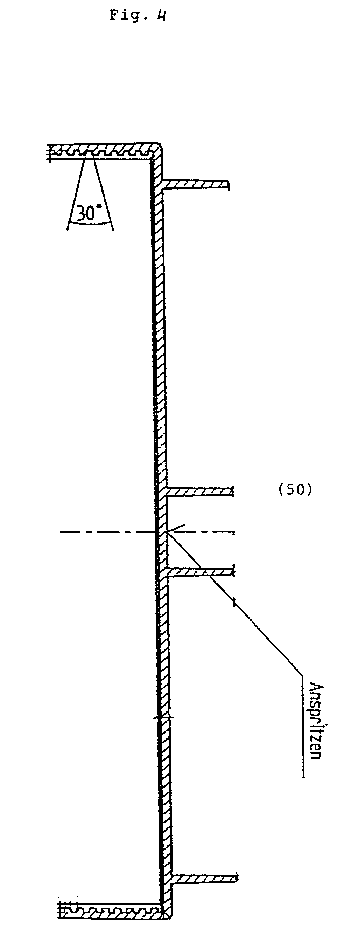

An der geöffneten Seite befindet sich ein stabiles Außengewinde 24; 34; 44; für das Verschrauben mit einem ebenfalls massiven Schraubdeckel 50; 100;. Der Deckel 100 und der Produktbehälterboden 23; 42; sind mit einem stabilen Haltegriff ausgeformt, so daß der Produktbehälter 20; 30; 40; einfach hantiert, transportiert und geöffnet werden kann. Gleichzeitig ist ein solider Verschluß 50; 100 (vgl. Fig. 4) für die Lager- und Transportsicherheit gegeben.

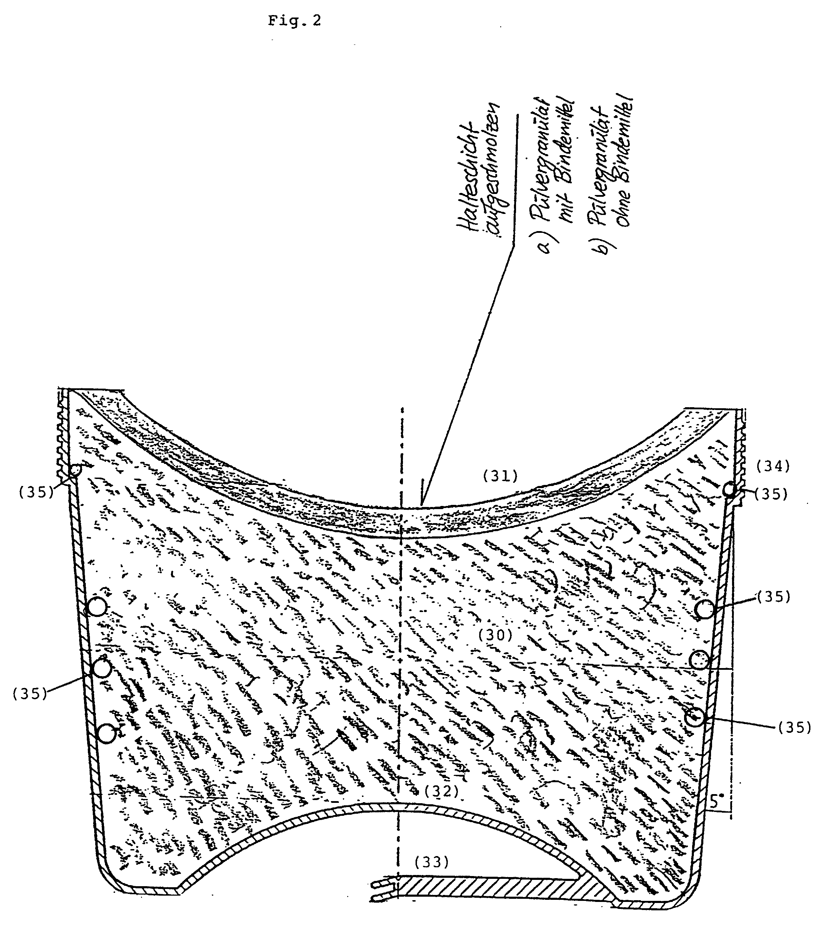

In einer besonderen Ausführungsform können an der Innenseite der Behälterwandung ein oder mehrere Halter 35 angebracht sein (vgl. Fig.2)

Pulverförmig und granulatförmige Füllinhalte können entweder in herkömmlicher Weise aus dem erfindungsgemäßen Behälter entnommen werden, oder aber, sofern bei der Abfüllung die Oberfläche in geeigneter Weise gegen das Herausrieseln abgeschlossen ist, durch Einstellen mit der Behälteröffnung nach unten in eine mit einem Siebeinsatz 81 ausgerüsteten Einspülvorrichtung 80 mittels gesteuerten, pulsierenden Bespülens der Granulatoberfläche 82 abgelöst und in das System eindosiert werden .

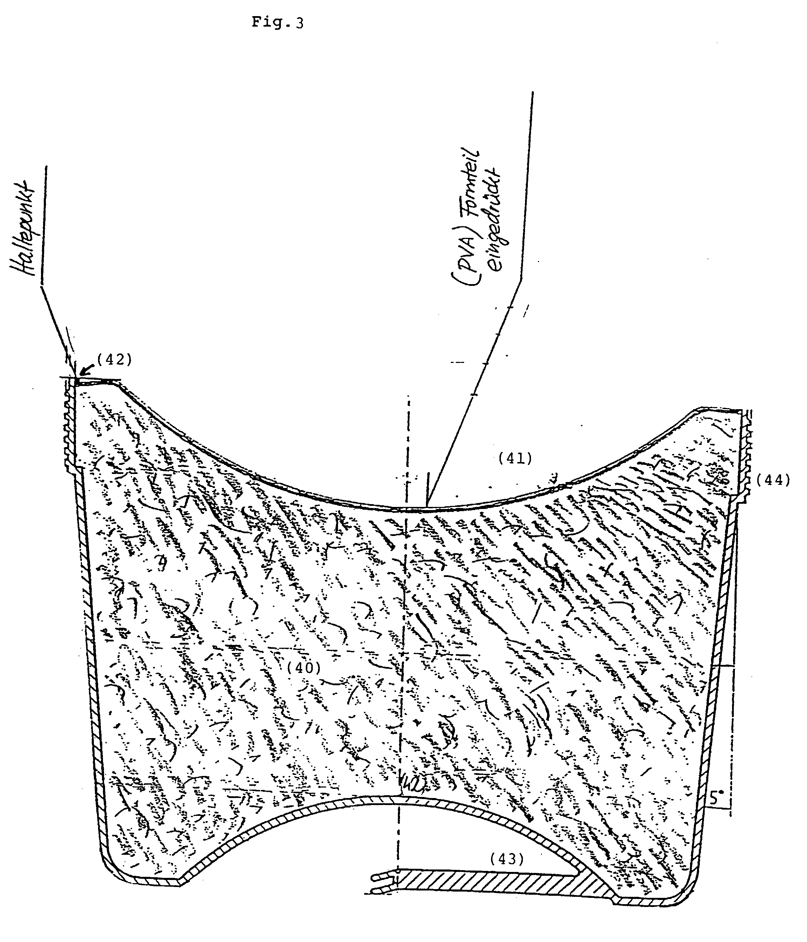

Als Verschlußprinzip zur Verhinderung des Herausrieselns beim Umdrehen der Behälter 20; 30; 40 und Einstellen mit der Öffnung nach unten in die Einspülvorrichtung 80 kann hierbei dienen:
(a) Befüllen des Behälters 20; 30; 40 und abschließendes Verschließen mit einer nicht wasserlöslichen Folie, der beispielsweise mit einer PE-Haltefolie 21 verschlossen werden kann (vgl. Fig.1), welche in der Einspülvorrichtung 80 aufgeschlitzt wird, so daß der Füllinhalt erst in der Einspülvorrichtung herausrieseln kann.
(b) Der Behälter 40 wird mit einer wasserlöslichen Folie 41 verschlossen, welche durch Heißsiegeln, Aufkleben, mechanisches Befestigen über der Behälteröffnung aufgebracht wird und beim Transport durch den aufgeschraubten Schraubdeckel gestützt wird. Beim Einsetzen in eine Dosiereinrichtung mit der Öffnung nach unten und Bespülen der Folie 41 mit Wasser, ggf. unter Erwärmen, löst sich diese in kurzer Zeit auf, so daß der pulver- oder granulatförmige Inhalt auf den Siebeinsatz rieseln kann.
(c) Der Behälter 30 wird mit dem Waschmittel mit und ohne Bindemittel befüllt. In einer Ausführungsform gemäß Fig. 2 wird der Behälterinhalt mit einem kalottenförmigen Preßstempel geformt und ggf. verdichtet; in einer anderen, hier nicht durch eine eigene Zeichnung dargestellten Ausführungsform kann auch eine ebene Oberfläche erzeugt werden. Anschließend wird eine Halteschicht aus einer wasserlöslichen Masse aufgebracht; diese Masse kann auch einer hochviskosen, erwärmten Schmelze, einer vorzugsweise hochviskosen verfestigungsfähigen Lösung oder einer verfestigungsfähigen Pulversustanz bestehen.
   In einer anderen Ausführungsform wird das Produkt mit oder ohne Zusatz von Bindemittel in den Behälter gefüllt und ein wasserlösliches PVA-Formteil 41 aufgebracht mit anschließendem Verpressen durch eine kalottenförmige Presse. Zusätzliche formschlüssige Haltepunkte 42 können am Produktbehälter 40 angebracht (vgl. Fig. 3).

Dieses vorerwähnte Beschichten des Pulvers im Behälter 20; 30; 40 mit einer wasserlöslichen, bei Temperaturen zwischen etwa 0°C und etwa 40°C festen Masse, geschieht auf die Weise, daß man eine fest an der Behälterwandung anliegende und haftende Schicht mit einer geeigneten Stärke (von beispielsweise 1 bis 2 cm) bildet, welche verhindert, daß der Füllinhalt beim Umstülpen und Einsetzen in die Einspülvorrichtung 80 herausrieselt. Hinsichtlich der chemischen Zusammensetzung muß diese Masse natürlich mit den Füllinhalten verträglich sein. Es kann sich - je nach Füllinhalt - z.B. um ein bei 0 bis 40°C festes Polyethylenglykol oder ein endgruppenverschlossenes Derivat davon handeln oder um eine kristallisationsfähige bzw. bei 0 bis 40°C sich verfestigende Lösung oder Schmelze von einem oder mehreren Salzen wie Soda, Natriumsulfat, Alkalipolyphosphat oder auch um Säuren wie Zitronensäure, handeln. Diese "schichtbildende" Substanz wird vorzugsweise so ausgewählt, daß bei eventuell zufälliger Berührung mit der Haut beim Manipulieren des Behälters der Kontakt keine negativen Folgen wie Reizung, Ätzung auf die Haut ausübt.

Wie in Fig. 8 gezeigt ist, besteht die Einspülvorrichtung 80 aus einem festverschließbaren Behälter 81 mit einem auf einem Sockel 82 ruhenden Dosiersieb 81, das zur Aufnahme der zu lösenden, sich in einem Produktbehälter 20; 30; 40 befindlichen Substanzen bestimmt ist, einem konzentrisch unter dem Sieb 81 im Behälterablaufteil 83 angebrachten Brause 84 und einem konzentrisch im Ablaufteil 83 angebrachten Ablaufstutzen 85. Die Befüllung der Dosiereinrichtung wird wie folgt durchgeführt. Die jeweiligen Schraubdeckel werden durch Aufschrauben vom jeweiligen Produktbehälter 20; 30; 40 entfernt. Die enthaltenen Spül- oder Waschmittel können aufgrund der angebrachten Haltefolie bzw. Halte schicht oder aufgrund des Halteformteils nicht herausrieseln, sondern sie bleiben dicht und geschützt im Behälter. Mit der Öffnung nach unten wird der Produktbehälter 20; 30; 40 in ein maßlich angepaßtes Dosiergerät 80 eingeführt und dort auf ein Dosiersieb 81 aufgesetzt (vgl. Fig. 8). Das Dosiersieb 81 kann die verschiedensten Formen aufweisen. In einer Ausführungsform ist es kalottenförmig. Der Deckel 86 des Dosiergerätes 80 wird geschlossen. Im Dosiergerät 80 werden dann bei angeforderter Dosierung per Leitfähigkeitssteuerung zunächst die wasserlöslichen Halteelemente (Folie, Halteschicht oder Halteformteil) abgelöst und dann das Pulvergranulat durch Aufsprühen einer wäßrigen Flüssigkeit aus der Brause 84 abgelöst. Das Dosiergerät 80 kann stufenlos gesteuert werden, so daß so lange Pulvergranulat aus dem Produktbehälter 20; 30; 40 abgegeben wird, bis kein Reinigung bzw. Waschmittel mehr vorhanden ist. Danach gibt das Steuergerät Alarm (optisch und akustisch) und das Bedienungspersonal entnimmt den geleerten Produktbehälter und setzt einen geöffneten, befüllten neuen Produktbehälter ein.

## Patentansprüche

1. Verfahren zum Herstellen verpreßter, geformter und wenigstens teilweise porös ausgebildeter Wasch-, Reinigungs-, Desinfektions- und/oder Konservierungsmittel ohne Verwendung einer vorerwärmten Schmelze oder Lösung dadurch gekennzeichnet, daß man eine Vormischung aus pulver- und/oder granulatförmigen Wirk- und Hilfssubstanzen mit Partikelgrößen von 0,01 bis 3mm herstellt, in einen recyclebaren, formstabilen Mehrwegbehälter mit einer Stapelschräge von 3 bis 7° und einer offenen, ausgerundeten Formgebung ohne Hinterschneidungen einfüllt und unter Anwendung von Druck zwischen 1 × 10⁴ bis 1 × 10⁶ Pa verdichtet, so daß ein in dem Behälter verfestigter Wasch-, Reinigungs-, Desinfektions- und/oder Konservierungsmittel-Block gebildet wird, bei welchemdie Pulverstruktur noch erkennbar ist.

2. Verfahren zum Herstellen verpreßter, geformter und wenigstens teilweise porös ausgebildeter Wasch-, Wasch-, Reinigungs-, Desinfektions- und/oder Konservierungsmittel ohne Verwendung einer vorerwärmten Schmelze oder Lösung dadurch gekennzeichnet,
- daß man eine vorgefertigte Mischung aus pulver- und/oder granulatförmigen Wirk- und Hilfsstoffen mit Partikelgrößen von 0,01 bis 3mm mit einem wasserlöslichen bzw. mit Wasser hydratisierbaren, bei der Herstellungstemperatur flüssigen bzw. hinreichend fließfähigen Bindemittel ausgewählt aus der Gruppe bestehend aus einer wässrigen Lösung oder einer Dispersion eines oder mehrerer wasselöslicher Salze oder einer organischen Substanz oder einer fließfähigen Schmelze oder Gels, wobei ggf. das Bindemittel selbst zwischen 0 und 40°C kristallisationsfähig ist in einer geeigneten Mischeinrichtung, vorzugsweise einem Durchlaufmischer, vermischt, so daß eine befeuchtete aber noch schüttfähige Pulvermasse entsteht,
- diese Pulvermasse in einen recyclebaren, formstabilen Mehrwegbehälter mit einer Stapelschräge von 3 bis 7° und einer offenen, ausgerundeten Formgebung ohne Hinterschneidungen einfüllt und
- unter Anwendung von Druck zwischen 1 × 10⁴ bis 1 × 10⁶ Pa verdichtet, so daß ein in dem Behälter verfestigter Wasch-, Reinigungs-, Desinfektions- und/oder Konservierungsmittel-Block gebildet wird, bei welchem die Pulverstruktur noch erkennbar ist.

3. Verfahren nach Anspruch 1 oder 2, dadurch gekennzeichnet, daß der verfestigte Wasch-, Reinigungs-, Desinfektions- und/oder Konservierungsmittel-Block mit einer Versiegelungsschicht aus einer aufgegossenen Lösung oder Schmelze einer wasserlöslichen bzw. in Wasser dispergierbaren Substanz versehen wird.

4. Verfahren gemäß irgendeinem der Ansprüche 1 und 2, dadurch gekennzeichnet, daß man zusätzlich eine Halte- oder Schutzschicht auf den Wasch-, Reinigungs-, Desinfektions- und/oder Konservierungsmittel-Block aufbringt.

5. Verfahren gemäß Anspruch 4, dadurch gekennzeichnet, daß die Schutz- oder Halteschicht aus einer nicht wasserlöslichen Folie besteht und diese ggf. mit dem Behälter verbunden wird.

6. Verfahren gemäß Anspruch 4, dadurch gekennzeichnet, daß man als Schutz- oder Halteschicht eine wasserlösliche Folie oder ein wasserlösliches Formteil verwendet, welche (welches) durch Heißsiegeln, Aufkleben, mechanisches Befestigen über der Behälteröffnung aufgebracht wird.

7. Verfahren gemäß Anspruch 4, dadurch gekennzeichnet, daß man den Wasch-, Reinigungs-, Desinfektions- und/oder Konservierungsmittel-Block mit einer Gußmasse beschichtet, die bei Temperaturen zwischen 0°C und 40°C fest ist.

8. Verfahren gemäß einem der vorhergehenden Ansprüche 1 bis 7, dadurch gekennzeichnet, daß man der Vormischung einen oder mehrere thermisch relativ instabile Wirkstoffe und/oder Zusätze, insbesonder Chlor- und/oder Sauerstoff liefernde Substanzen und/oder Duft- und/oder Farbstoff zusetzt.

9. Verfahren gemäß Anspruch 2, dadurch gekennzeichnet, daß das Bindemittel in Anteilen von 1 bis 29 Masse-%, vorzugsweise 2 bis 10 Masse-%, besonders bevorzugt zwischen 3 bis 8 Masse-%, bezogen auf die fertige Gesamtmischung, eingesetzt wird.

10. Verfahren gemäß Anspruch 10, dadurch gekennzeichnet, daß das Bindemittel selbst reinigungsaktiv wirkende Bestandteile, insbesondere eine wäßrige 30- bis 60-%ige Lösung von Alkali-(ortho-, pyro- oder poly-)phosphaten enthält.

11. Verfahren gemäß Anspruch 3 und 9 bis 1O, dadurch gekennzeichnet, daß man als Versiegelungsschicht eine Gußmasse gleicher oder verschiedener Zusammensetzung wie das Bindemittel einsetzt, wobei diese Gußmasse bei Temperaturen zwischen 0 und 40°C fest oder zumindest gelförmig ist.

12. Verfahren gemäß Anspruch 11, dadurch gekennzeichnet, daß der Anteil der Gußmasse, bezogen auf den gesamten Füllinhalt des Behälters zwischen 0 und maximal 20 Masse-% beträgt.

13. Verfahren gemäß Anspruch 11, dadurch gekennzeichnet, daß man als Gußmasse für die Versiegelungsschicht ein bei 0 bis 40°C festes Polyethylenglykol oder ein endgruppenverschlossenes Derivat desselben oder eine kristallisationsfähige, sich bei 0 bis 40°C verfestigende Lösung oder Schmelze von einem oder mehreren Salz(en) wie Soda, Natriumsulfat, Alkalipolyphosphat oder Säuren wie Zitronensäure oder Mischungen dieser Komponenten einsetzt.

14. Verfahren gemäß Anspruch 11 und 12, dadurch gekennzeichnet, daß man als Versiegelungsschicht eine Mischung aus 30 bis 50 Masse-% Wasser sowie 40 Masse-% Natriumpolyphosphat, und/oder 20 bis 50 Masse-% Soda und/oder 20 bis 50 Masse-% Natriumsulfat einsetzt, wobei entweder nur ein oder eine Kombination von zwei oder drei geeigneten Salz(en) enthalten ist (sind) und der Feststoffgehalt zwischen 50 und 70 Masse-% liegt.

15. Verfahren gemäß irgendeinem der vorhergehenden Ansprüche 1 bis 14, dadurch gekennzeichnet, daß man den Behälter mit einer mit ihm verbundenen Abdeckung versieht.

16. Verfahren gemäß einem der vorhergehenden Ansprüche 1 bis 15, dadurch gekennzeichnet, daß man die in den Behälter eingefüllte Pulvermasse unter Anwendung eines Drucks von 1x10⁴ - 1x10⁶ Pa (0,1 bis 10 at) mit einem ebenen Stempel verpreßt und ggf. anschließend beschichtet, versiegelt oder mit einem Formteil oder einer Folie versieht.

17. Verfahren gemäß einem der vorhergehenden Ansprüche 1 bis 16, dadurch gekennzeichnet, daß man die in den Behälter eingefüllte Pulvermasse unter Anwendung eines Drucks von 1x10⁴ - 1x10⁶ Pa (0,1 bis 10 at) mit einem kalottenförmigen Stempel verpreßt und ggf. anschließend beschichtet, versiegelt oder mit einem Formteil oder einer Folie versieht..

18. Recyclebarer, insbesondere zum Einsatz in Spül- und Waschmaschinen geeigneter Wasch-, Reinigungs-, Desinfektions- und/oder Konservierungsmittel enthaltender Mehrweg-Behälter (20; 30; 40) , der derart gestaltet ist, daß Wasch-, Reinigungs-, Desinfektions- und/oder Konservierungsmittel aus dem Behälter abgegeben wird, wenn ein Strom wäßriger Flüssigkeit auf eine diesem ausgesetzte Oberfläche des Wasch-, Reinigungs-, Desinfektions- und/oder Konservierungsmittel gegeben wird, dadurch gekennzeichnet, daß das Wasch-, Reinigungs-, Desinfektions- und/oder Konservierungsmittel gemäß dem Verfahren nach einem der Ansprüche 1 bis 17 erhältlich ist.

19. Mehrweg-Behälter gemäß Anspruch 18, dadurch gekennzeichnet, daß der Boden (23; 32; 42) des Behälters vertieft, gegebenenfalls kalottenförmig ausgestaltet ist und in diesen Boden hineinragenden einen Haltegriff (22, 33, 43) zum Hantieren und Transport des Behälters aufweist.

20. Mehrweg-Behälter gemäß Anspruch 19, dadurch gekennzeichnet, daß an der geöffneten Seite des Behälters ein Außengewinde (24; 34; 44) für das Verschrauben oder Anbringen mit einem Schraubdeckel oder einer anderen formschlüssigen Verbindung, gegebenenfalls einem Bajonettverschluß vorgesehen ist.

21. Mehrweg-Behälter gemäß Anspruch 18 und 19, dadurch gekennzeichnet, daß an der Innenseite der Wandung des Behälters ein oder mehrere Halter (35) angebracht (ist) sind.

22. Mehrweg-Behälter gemäß Anspruch 18 bis 21, dadurch gekennzeichnet, daß er eine Stapelschräge von 5° aufweist.

23. Verwendung eines Wasch-, Reinigungs-, Desinfektions- und/oder Konservierungsmittels-Mehrweg-Behälters gemäß einem der Ansprüche 18 bis 22 zum Waschen, Reinigen, Spülen und/oder Desinfizieren und/oder zur antimikrobiellen bzw. deodorierenden Behandlung von Wasserkreisläufen und/oder von wasserführenden Systemen.

## Claims

1. A process for the production of compacted, shaped and at least partly porous detergents, cleaners, disinfectants and/or preservatives without using a preheated melt or solution, characterized in that a compound of powder-form and/or granular active substances and auxiliaries with particle sizes of 0.01 to 3 mm is prepared, introduced into a recyclable, dimensionally stable returnable container with a stacking slope of 3 to 7° and an open, rounded shape with no undercuts and compacted under a pressure of 1 x 10⁴ to 1 x 10⁶ Pa so that a solid block of detergent, cleaner, disinfectant and/or preservative in which the powder structure is still discernible is formed in the container.

2. A process for the production of compacted, shaped and at least partly porous detergents, cleaners, disinfectants and/or preservatives without using a preheated melt or solution, characterized in that
- a prepared mixture of powder-form and/or granular active substances and auxiliaries with particle sizes of 0.01 to 3 mm is mixed in a suitable mixer, preferably a continuous mixer, with a water-soluble or water-hydratable binder which is liquid or sufficiently flowable at the production temperature, being selected from the group consisting of an aqueous solution or a dispersion of one or more water-soluble salts or an organic substance or a flowable melt or gel, the binder itself optionally being crystallizable between 0 and 40°C, so that a moistened but still pourable powder is formed,
- the powder is introduced into a recyclable, dimensionally stable returnable container with a stacking slope of 3 to 7° and an open, rounded shape with no undercuts and
- is compacted under a pressure of 1 x 10⁴ to 1 x 10⁶ Pa so that a solid block of detergent, cleaner, disinfectant and/or preservative in which the powder structure is still discernible is formed in the container.

3. A process as claimed in claim 1 or 2, characterized in that the solid block of detergent, cleaner, disinfectant and/or preservative is provided with a sealing layer of a poured-on solution or melt of a water-soluble or water-dispersible substance.

4. A process as claimed in claim 1 or 2, characterized in that a holding or protective layer is additionally applied to the block of detergent, cleaner, disinfectant and/or preservative.

5. A process as claimed in claim 4, characterized in that the protective or holding layer consists of a water-insoluble film which is optionally joined to the container.

6. A process as claimed in claim 4, characterized in that the protective or holding layer is a water-soluble film or a water-soluble moulding which is applied over the opening of the container by heat-sealing, bonding or mechanical fastening.

7. A process as claimed in claim 4, characterized in that the block of detergent, cleaner, disinfectant and/or preservative is coated with a cast compound which is solid at temperatures of 0°C to 40°C.

8. A process as claimed in any of preceding claims 1 to 7, characterized in that one or more thermally relatively unstable active substances and/or additives, more particularly chlorine- and/or oxygen-yielding substances and/or perfume and/or dye, is/are added to the compound.

9. A process as claimed in claim 2, characterized in that the binder is used in quantities of 1 to 29% by weight, preferably in quantities of 2 to 10% by weight and more preferably in quantities of 3 to 8% by weight, based on the final mixture as a whole.

10. A process as claimed in claim 10, characterized in that the binder itself contains cleaning-active components, more particularly an aqueous 30 to 60% solution of alkali metal (ortho, pyro or poly)phosphates.

11. A process as claimed in claims 3 and 9 to 10, characterized in that the sealing layer is a cast compound with the same composition as, or differing in composition from, the binder, this cast compound being solid or at least gel-like at temperatures of 0 to 40°C.

12. A process as claimed in claim 11, characterized in that the cast compound makes up 0 to at most 20% by weight of the total capacity of the container.

13. A process as claimed in claim 11, characterized in that the cast compound of the sealing layer is a polyethylene glycol solid at 0 to 40°C or an end-capped derivative thereof or a crystallizable solution or melt - solidifying at 0 to 40°C - of one or more salts(s), such as soda, sodium sulfate, alkali metal polyphosphate, or acids, such as citric acid, or mixtures of these components.

14. A process as claimed in claims 11 and 12, characterized in that the sealing layer consists of a mixture of 30 to 50% by weight of water and 40% by weight of sodium polyphosphate and/or 20 to 50% by weight of soda and/or 20 to 50% by weight of sodium sulfate, either only one suitable salt or a combination of two or three suitable salt(s) being present and the solids content being between 50 and 70% by weight.

15. A process as claimed in any of preceding claims 1 to 14, characterized in that the container is provided with a cover which is connected to it.

16. A process as claimed in any of preceding claims 1 to 15, characterized in that the powder introduced into the container is compacted under a pressure of 1 x 10⁴ to 1 x 10⁶ Pa (0.1 to 10 atms) using a flat stamp and is then optionally coated, sealed or provided with a moulding or a film.

17. A process as claimed in any of preceding claims 1 to 16, characterized in that the powder introduced into the container is compacted under a pressure of 1 x 10⁴ to 1 x 10⁶ Pa (0.1 to 10 atms) using a hemispherical stamp and is then optionally coated, sealed or provided with a moulding or a film.

18. A recyclable returnable container (20;30;40) containing detergent, cleaner, disinfectant and/or preservative which is suitable for use in washing and dishwashing machines and which is designed in such a way that detergent, cleaner, disinfectant and/or preservative is dispensed from the container when a stream of aqueous liquid impinges on an exposed surface of the detergent, cleaner, disinfectant and/or preservative, characterized in that the detergent, cleaner, disinfectant and/or preservative is obtainable by the process claimed in any of claims 1 to 17.

19. A retumable container as claimed in claim 18, characterized in that the base (23;32;42) of the container is recessed and optionally hemispherical in shape and comprises a handle (22,33,43) which projects into the base for handling and carrying the container.

20. A returnable container as claimed in claim 19, characterized in that an external screwthread (24;34;44) is provided on the open side of the container for screwing on or applying a screw top or another positive connection, optionally a bayonet closure.

21. A returnable container as claimed in claim 18 or 19, characterized in that one or more holders (35) is/are provided on the inside of the wall of the container.

22. A retumable container as claimed in claims 18 to 21, characterized in that it has a 5° stacking slope.

23. The use of the returnable detergent, cleaner, disinfectant and/or preservative container claimed in any of claims 18 to 22 for washing, cleaning, dishwashing and/or disinfecting and/or for the antimicrobial or deodorizing treatment of water circuits and/or water-carrying systems.

## Revendications

1. Procédé de fabrication d'un produit de lavage, de nettoyage, de désinfection et/ou de conservation comprimé, moulé et de structure au moins partiellement poreuse, sans utilisation d'une masse fondue préchauffée ou d'une solution,
caractérisé en ce qu'
on prépare un prémélange de principes actifs et d'adjuvants en poudre et/ou granulés ayant des tailles de particules de 0,01 à 3 mm, on les conditionne dans des récipients réutilisables, de forme stable, recyclables ayant une inclinaison d'emboîtement de 3 à 7° et une forme arrondie ouverte sans contredépouilles et on densifie en utilisant la pression entre 1x10⁴ et 1 x 10⁶ Pa de façon à former un bloc de produit de lavage, de nettoyage, de désinfection et/ou de conservation solidifié dans le récipient, ou on reconnaît encore la structure de poudre.

2. Procédé de préparation d'un produit de lavage, de nettoyage, de désinfection et/ou de conservation comprimé, moulé et de structure au moins partiellement poreuse, sans utilisation d'une masse fondue préchauffée ou d'une solution,
caractérisé en ce qu'
- on utilise un mélange préparé au préalable de principes actifs et d'adjuvants en poudre et/ou en granulés, ayant des tailles de particules comprises entre 0,01 et 3 mm qu'on mélange à un liant liquide ou suffisamment coulable à la température de préparation, soluble ou hydratable à l'eau, dans un dispositif de mélange adapté de préférence un mélangeur en ligne, choisi dans le groupe constitué d'une solution aqueuse ou d'une dispersion d'un ou plusieurs sels solubles dans l'eau, ou d'une substance organique ou d'une masse fondue coulable ou d'un gel, où le cas échéant le liant lui-même peut cristalliser entre 0 et 40°C, de façon à former une masse de poudre humidifiée mais encore coulable.
- On conditionne cette masse de poudre dans des récipients réutilisables, recyclables de forme stable, ayant une inclinaison d'emboîtement de 3 à 7° et une forme arrondie ouverte sans contredépouilles,
- et on densifie en utilisant la pression entre 1 x 10⁴ et 1 x 10⁶ Pa de façon à former un bloc de produit de lavage, de nettoyage, de désinfection et/ou de conservation solidifié dans le récipient ou on reconnaît encore la structure de la poudre.

3. Procédé selon la revendication 1 ou 2,
caractérisé en ce que
le bloc de produit de lavage, de nettoyage, de désinfection et/ou de conservation solidifié est muni d'une couche de scellement obtenue à partir d'une solution ou d'une masse fondue coulable d'une substance hydrosoluble ou dispersible dans l'eau.

4. Procédé selon l'une quelconque des revendications 1 et 2,
caractérisé en ce qu'
on dépose en plus une couche de maintien ou de protection sur le bloc de lavage, de nettoyage, de désinfection et/ou de conservation.

5. Procédé selon la revendication 4,
caractérisé en ce que
la couche de maintien ou de protection est en film non hydrosoluble et est lié au récipient le cas échéant.

6. Procédé selon la revendication 4,
caractérisé en ce qu'
on utilise comme couche de maintien ou de protection un film hydrosoluble ou une pièce moulée hydrosoluble qu'on dépose par thermoscellage, collage, fixation mécanique sur l'orifice du récipient.

7. Procédé selon la revendication 4,
caractérisé en ce qu'
on recouvre le bloc de lavage, de nettoyage, de désinfection et/ou de conservation avec une masse coulée, qui est solide à des températures comprises entre 0°C et 40°C.

8. Procédé selon l'une des revendications précédentes 1 à 7,
caractérisé en ce qu'
on ajoute au prémélange une ou plusieurs substances actives et/ou des adjuvants thermiquement relativement instables, notamment des substances libérant du chlore et/ou de l'oxygène et/ou un colorant ou un parfum.

9. Procédé selon la revendication 2,
caractérisé en ce qu'
on utilise le liant en des teneurs de 1 à 29 % en masse, de préférence 2 à 10 % en masse, surtout entre 3 et 8 % en masse, par rapport au mélange global terminé.

10. Procédé selon la revendication 9,
caractérisé en ce que
le liant lui-même contient des composants à effet de nettoyage, notamment une solution aqueuse de 30 à 60 % d' (ortho-, pyro- ou poly-)phosphates d'alcalin.

11. Procédé selon les revendications 3 et 9 à 10,
caractérisé en ce qu'
on utilise comme couche de scellement une masse à couler de composition identique ou différente à celle du liant, où cette masse à couler est solide entre 0 et 40°C ou au moins gélifiée.

12. Procédé selon la revendication 11,
caractérisé en ce que
la teneur en masse à couler, par rapport à l'ensemble du contenu du récipient représente entre 0 et 20 % en masse au maximum.

13. Procédé selon la revendication 11,
caractérisé en ce qu'
on utilise comme masse à couler pour la couche de scellement un polyéthylèneglycol solide entre 0 et 40°C ou un de ses dérivés à groupes terminaux bloqués, ou une solution ou une masse fondue pouvant cristalliser entre 0 et 40°C constituée d'un ou plusieurs sels comme le carbonate de sodium le sulfate de sodium, le polyphosphate d'alcalin ou des acides comme l'acide citrique ou des mélanges de ces composants.

14. Procédé selon la revendication 11 et 12,
caractérisé en ce qu'
on utilise comme couche de scellement un mélange de 30 à 50 % en masse d'eau ainsi que de 40 % en masse de polyphosphate de sodium et/ou de 20 à 50 % en masse de carbonate de sodium et/ou 20 à 50 % en masse de sulfate de sodium, où on utilise soit seulement un, soit une combinaison de deux ou trois sels appropriés et la teneur en solide est comprise entre 50 et 70 % en masse.

15. Procédé selon l'une quelconques des revendications précédentes 1 à 14,
caractérisé en ce qu'
on munit le récipient d'un couvercle amovible.

16. Procédé selon l'une des revendications précédentes 1 à 15,
caractérisé en ce qu'
on comprime la masse de poudre conditionnée dans le récipient en utilisant une pression de 1 x 10⁴ - 1 x 10⁶ Pa (0,1 à 10 at) avec un poinçon plan et ensuite le cas échéant on enduit, scelle ou on munit d'une pièce moulée ou d'un film.

17. Procédé selon l'une des revendications précédentes 1 à 16,
caractérisé en ce qu'
on comprime la masse de poudre chargée en utilisant une pression de 1 x 10⁴ - 1 x 10⁶ Pa (0,1 à 10 at) avec un poinçon en calotte et le cas échéant on enduit, scelle ou munit d'une pièce moulée ou d'un film.

18. Récipient (20, 30, 40) réutilisable recyclable contenant du produit approprié de lavage, de nettoyage, de désinfection et/ou de conservation à utiliser dans les machines à laver le linge ou la vaisselle qui est conformé de telle sorte que le produit de lavage, de nettoyage, de désinfection et/ou de conservation est relâché du récipient, quand un courant de liquide aqueux est dirigé sur une surface qui lui est opposée du produit de lavage, de nettoyage, de désinfection et/ou de conservation,
caractérisé en ce qu'
on prépare le produit de lavage, de nettoyage, de désinfection et/ou de conservation selon le procédé suivant l'une des revendications 1 à 17.

19. Récipient réutilisable selon la revendication 8,
caractérisé en ce que
le fond (23, 32, 42) du récipient est évidé le cas échéant est en forme de calotte et qu'il présente une poignée plongeant dans ce fond (22, 33, 43) pour manipuler et transporter le récipient.

20. Récipient réutilisable selon la revendication 19,
caractérisé en ce que
le côté ouvert du récipient présente un filetage extérieur pour le vissage ou la pose d'un couvercle à vis ou toute autre liaison par emboîtement de forme, le cas échéant une fermeture à baïonnette.

21. Récipient réutilisable selon la revendication 18 et 19,
caractérisé en ce que
sur la paroi intérieure du récipient on dispose un ou plusieurs supports (35).

22. Récipient réutilisable selon les revendications 18 à 21,
caractérisé en ce qu'
il présente une pente d'emboîtement de 5°.

23. Utilisation d'un récipient réutilisable d'un produit de lavage, de nettoyage, de désinfection et/ou de conservation selon l'une des revendications 18 à 22 pour laver, nettoyer, laver la vaisselle et/ou désinfecter et/ou pour le traitement antimicrobien ou désodorisant de circuits d'eau et/ou de systèmes de conduites d'eau.
